(19) Europäisches Patentamt
European Patent Office
Office européen des brevets

(11) Veröffentlichungsnummer : **0 122 909**
**B1**

(12) # EUROPÄISCHE PATENTSCHRIFT

(45) Veröffentlichungstag der Patentschrift : **30.09.87**

(51) Int. Cl.⁴ : **C 07 K 15/06**, A 61 K 39/395

(21) Anmeldenummer : **84890050.2**

(22) Anmeldetag : **15.03.84**

(54) **Immunglobulin-G-hältige Fraktion.**

(30) Priorität : **16.03.83 AT 929/83**
**16.03.83 AT 930/83**

(43) Veröffentlichungstag der Anmeldung : **24.10.84 Patentblatt 84/43**

(45) Bekanntmachung des Hinweises auf die Patenterteilung : **30.09.87 Patentblatt 87/40**

(84) Benannte Vertragsstaaten : **AT BE CH DE FR GB IT LI NL SE**

(56) Entgegenhaltungen :
**DE-A- 2 856 939**
**DE-A- 2 936 047**

(73) Patentinhaber : **IMMUNO Aktiengesellschaft für chemisch-medizinische Produkte**
**Industriestrasse 72**
**A-1220 Wien (AT)**

(72) Erfinder : **Eibl, Johann, Dr.**
**Gustav Tschermakgasse 2**
**A-1180 Wien (AT)**
Erfinder : **Linnau, Yendra, Dr.**
**Lavendelweg 24**
**A-1224 Wien (AT)**
Erfinder : **Schwarz, Otto, Dr.**
**Celtesgasse 5**
**A-1190 Wien (AT)**

(74) Vertreter : **Wolfram, Gustav, Dipl.-Ing.**
**Schwindgasse 7 P.O. Box 205**
**A-1041 Wien (AT)**

**EP 0 122 909 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft eine Immunglobulin-G-hältige Fraktion aus menschlichem oder tierischem Plasma zur intravenösen Anwendung.

Immunglobulinhältige Präparate können bei primären und sekundären Immundefekten, A- oder Hypogammaglobulinamie, bei Antikörpermangelsyndrom, bei Virusinfektionen oder bakteriellen Infektionen angewendet werden.

Zur Gewinnung von immunoglobulinhältigen Präparationen aus menschlichem oder tierischem Plasma sind bereits verschiedene Methoden bekannt, so z. B. die Ausfällung mit Äthanol (J. L. Oncley, M. Melin, D. A. Richart, J. W. Cameron und P. M. Gross, J. Am. Chem. Soc. 71, 541 (1949)) sowie modifizierte Äthanolverfahren nach H. F. Deutsch, L. J. Gosting, R. A. Alberty und J. W. Williams, J. Biol. Chem. 164, 109 (1964), sowie P. Kistler und H. Nitschman, Vox Sanguinis 7, 414 (1962).

Weiters ist eine Methode bekannt, nach der Immunglobulin aus Plasma mittels Ammonsulfat und Polyäthylenglykol gefällt wird (A. Polson, G. M. Potgieter, J. F. Largrier, G.E.F. Mears und F. J. Jourbet, Biochim. Biophys. Acta. 82, 463 (1964)). Nach anderen Verfahren wurde die Anwendung von Ionenaustauschern vorgeschlagen (E. A. Peterson und H. A. Sober, J. Am. Chem. Soc. 78, 751 (1956)).

Diese Methoden hatten den Nachteil, daß die gewonnenen Präparate lediglich für eine intramuskuläre Applikation geeignet waren. Bei intravenöse Anwendung zeigten sie unerwünschte Nebenreaktionen, wie eine vasoaktive Wirkung.

Man hat sich daher bemüht, Nebenerscheinungen bzw. Nebenwirkungen zu verringern, zu welchem Zweck immunglobulinhältige Präparate mit löslichen proteolytischen Enzymen, wie Pepsin, Plasmin, Papain u. a. behandelt wurden (DE-PS 1 148 037). Bei dieser Behandlung wird jedoch die Molekülstruktur der Immunglobuline verändert, mit der Folge einer verkürzten biologischen Halbwertszeit. Es wurde auch festgestellt, daß Enzymreste in den Präparaten zurückbleiben, wodurch diese kontaminiert werden. Die Lagerstabilität ist dementsprechend gering, die Gefahr des Fortschreitens der proteolytischen Spaltung groß.

In der DE-OS 29 36 047 ist ein Verfahren zur Herstellung eines intravenös verabreichbaren Immunglobulinpräparates beschrieben, bei welchem eine kombinierte Reinigung mit Ammoniumsulfat und Polyäthylenglykol in Gegenwart eines löslichen Kohlehydrates oder eines Polyols vorgenommen wird. Wenngleich damit vasoaktive Nebenerscheinungen vermieden werden konnten, so bleibt eine Verbesserung in bezug auf die Sicherheit und Reproduzierbarkeit bei intravenöser Anwendung weiterhin wünschenswert.

Zum Stand der Technik sind auch die japanischen Patentschriften JP-OS 56-7721 und JP-OS 56-15215 sowie die DE-OS 32 20 309 zu zählen, die Verfahren zur Herstellung von intravenös applizierbaren Immunglobulin-Präparaten zum Ziel haben. Die Behandlung soll mit immobilisiertem Plasmin bzw. immobilisiertem Pepsin erfolgen, doch sind auch die hiermit erreichbaren Ergebnisse nicht zufriedenstellend, weil die Präparate eine unerwünscht hohe antikomplementäre Aktivität aufweisen.

In der Ep-A-00 35 616 ist weiterhin ein Verfahren zur Herstellung einer Immunoglobulin-G-hältigen Fraktion beschrieben, die hauptsächlich IgG-Monomere enthält.

Die Erfindung bezweckt die Vermeidung der geschilderten Nachteile und Schwierigkeiten und stellt sich die Aufgabe, eine immunglobulinhältige Fraktion aus menschlichem oder tierischem Plasma zu schaffen, die intravenös applizierbar und in optimaler Weise nebenwirkungsfrei ist. Diese Freiheit soll sich auch auf unerwünschte leukopenische und bronchospastische Nebenwirkungen erstrecken. Weiters soll die erfindungsgemäße Immunglobulin-G-hältige Fraktion eine sehr geringe antikomplementäre Aktivität aufweisen.

Diese Aufgabe wird erfindungsgemäß dadurch gelöst, daß die Fraktion

mindestens 90 % monomere IgG-Moleküle,

mindestens 95 % Gammaglobuline,

mindestens 90 % funktionell intakte IgG-Moleküle, d. h. mindestens 90 % IgG-Moleküle, die an Protein A von Staphylococcus aureus bindbar sind, enthält, eine antikomplementäre Aktivität von mindestens 40 mg Protein zur Neutralisation einer C'H-50-Einheit aufweist und im wesentlichen frei ist von vasoaktiv und leukopenisch wirksamen sowie von bronchospastischen Substanzen, ausgedrückt durch folgende Merkmale :

a) daß die vasoaktive Wirkung im Hundetest als Durchschnitt bei vier Tieren, d. h. ein Blutdruckabfall von höchstens 30 %, erst bei einer Dosis von mehr als 500 mg/kg Körpergewicht,

b) daß die leukopenische Wirkung im Hundetest als Durchschnitt bei vier Tieren, d. h. ein Abfall der Leukozytenzahl von höchstens 50 %, erst bei einer Dosis von mehr als 500 mg/kg Körpergewicht, und

c) daß die bronchospastische Wirkung im Meerschweinchentest als Durchschnitt bei vier Tieren, d. h. ein Atemdruckanstieg von höchstens 30 %, erst bei einer Dosis von mehr als 500 mg/kg Körpergewicht feststellbar ist.

Die Bestimmung der funktionell intakten IgG-Moleküle erfolgt nach der Protein-A-Sepharose Methode (FEBS Letters, Vol. 28, 1972, 73 ff. ; H. Hjelm, K. Hjelm, J. Sjöquist, « Protein A from staphylococcus aureus, its solution by affinity chromatography and its use as an immunosorbent for isolation of immunoglobulins »). Die Methode beruht darauf, daß das Protein A des Staphylococcus aureus mit den IgG-Molekülen der Subklassen IgG 1, 2 und 4 in Wechselwirkung tritt und sie bindet. Die funktionell aktiven Stellen sind die $CH_2$ und $CH_3$-Regionen, das sind Teile der H-Kette der IgG-Moleküle.

Die vereinigten Fraktionen $V_e/V_0$ von 1,30 bis 2,20 aus der Molekulargewichtsbestimmung mittels Gelfiltration (Diagramm I) werden auf eine bestimmte Proteinkonzentration eingestellt und 10 mg Protein dieser Präparation über 10 ml Protein A-Sepharose, immobilisiertes Protein A, chromatographiert. Die gebundenen IgG 1, 2 und 4 werden mit einem Natriumcitrat-Citronensäure-Puffer, pH 3,0, eluiert. Dann wird das gebundene und ungebundene IgG errechnet.

Die erfindungsgemäße Fraktion weist eine so geringe antikomplementäre Wirkung auf, daß sie nicht weniger als 40 mg Protein zur Neutralisation einer C'H50-Einheit benötigt.

Die Bestimmung dieses Merkmales erfolgt gemäß « Public Health Monograph » Nr. 74 ; Standardized diagnostic complement fixation method and adaptation to microtest, Washington, 1965, und E. A. Kabal und M. Mayer, Experimental immunochemistry ; 2nd ed. Thomas Springfield 1961.

Bei elektrophoretischer Bestimmung findet man bei den erfindungsgemäßen Fraktionen mindestens 95 % Gammaglobulin. Die Bestimmung erfolgt gemäß Michael D. Gebott, Beckman Microzone Elektrophoresis Manual, Beckman Instruments, Inc. 1977, 015-083630-C.

Charakteristisch für die erfindungsgemäßen Immunglobulin-G-hältigen Fraktionen sind ihre pharmakologischen Eigenschaften : sie sind nämlich im wesentlichen frei von vasoaktiv und leukopenisch wirksamen sowie von bronchospastischen Substanzen.

Die vasoaktive Wirkung wird in folgender Weise festgestellt :

Versuchstieren (Mischlingen beiderlei Geschlechts) wird nach Narkotisierung die Vena jugularis und die Arterie carotis präpariert. Vor der Anästhesie wird eine Fastenzeit von mindestens 12 Stunden angesetzt. Pro Testsubstanz werden je vier qualifizierte Hunde, d. h. solche Tiere benötigt, die bei intraarterieller Applizierung von standardisiertem intramuskulär anwendbarem Immunglobulin (« Standardsubstanz ») eine vasoaktive Wirkung (Blutdruckabfall) von mindestens 30 % bei einer Dosierung von 50 mg/kg Körpergewicht zeigen. Dieses standardisierte intramuskulär anwendbare Immunglobulin wird nach der einleitend erwähnten Methode von J. L. Oncley, M. Melin, D. A. Richart, J. W. Cameron und P. M. Gross, J. Am. Chem. Soc. 71, 541 (1949) bereitet. Hunde, die auf die Standardsubstanz keine Reaktion zeigen, können für die Vergleichstests nicht herangezogen werden.

Von der Standardsubstanz werden 160 mg in 1 ml Aqua ad iniectabilia gelöst und mit isotoner NaCl-Lösung auf 16,7 mg/ml verdünnt. Das aufgelöste Material wird innerhalb von vier Stunden verwendet.

Das erfindungsgemäße intravenös anwendbare Immunglobulin-G wird mit Aqua ad iniectabilia so gelöst, daß 1 ml 165 mg Protein enthält. Das aufgelöste Material wird innerhalb von vier Stunden verwendet.

Die Tiere werden mit einer intravenösen Einzeldosis von 40 mg/kg Nembutal (Barbitural) anästhesiert, und die Vena jugularis externa wird nach ihrer Aufteilung am unteren Rand des Unterkiefers präpariert und ein Katheter eingebunden. Danach wird die Arterie carotis vom gleichen Hautschnitt aus freigelegt und ein Katheter eingebunden. Nach der Präparation der Arterie wird 30 min zugewartet, um stabile Ausgangswerte zu erreichen. Über den tiefen Venenkatheter wird der zentrale Venendruck und über den seichten arteriellen Katheter wird der arterielle Blutdruck mit Hilfe eines Drucktransducers gemessen. Über den arteriellen Katheter wird zuerst das erfindungsgemäß iv. anwendbare Immunglobulin-G und dann die Standardsubstanz injiziert.

Während der gesamten Zeit der Versuchsdurchführung wird über den Arterienkatheter der systolische und diastolische Blutdruck mit Hilfe des Drucktransducers aufgezeichnet.

Der Blutdruckmittelwert (systolisch und diastolisch) sowie der Mittelwert der Leukozytenzahl vor der Injektion der Testsubstanz und der Standardsubstanz werden bestimmt. Der maximale Blutdruckabfall wird durch Messung des Blutdruckes über 20 min nach Injektion der Testsubstanzen bestimmt.

Die vasoaktive Wirkung von erfindungsgemäß iv. anwendbarem Immunglobulin-G wird so ermittelt, daß 500 mg/kg Körpergewicht Hund injiziert werden und der durchschnittliche prozentuelle systolische und diastolische Blutdruckabfall bei vier Hunden mit der blutdrucksenkenden Wirkung von im. anwendbarer Standardsubstanz verglichen wird.

Die Bestimmung der leukopenischen Wirkung wird in folgender Weise durchgeführt :

Versuchstieren (Mischlingshunden beiderlei Geschlechts) wird nach Narkotisierung die Vena jugularis und die Arterie carotis präpariert. Vor der Anästhesie wird eine Fastenzeit von mindestens 12 Stunden angesetzt. Pro Testsubstanz werden je vier qualifizierte Hunde benötigt, die bei intraarterieller Applizierung von standardisiertem intramuskulär anwendbarem Immunglobulin (Standardsubstanz) eine leukopenische Wirkung (Leukozytenabfall) von mindestens 50 % bei einer Dosierung von 50 mg/kg Körpergewicht zeigen. Hunde, die auf die Standardsubstanz keine Reaktion zeigen, können für die Vergleichstests nicht herangezogen werden.

Die Vorbereitung der Versuchstiere und der Testsubstanzen erfolgt in gleicher Weise wie vorstehend beschrieben.

Zur Untersuchung der Leukozytenzahl werden Blutproben gezogen. Bei der ersten Blutprobe werden 40 µl Blut mit 20 ml Isotone II® (Coulter) und sechs Tropfen Zapoglobin® (Coulter) versetzt und im Coulter Counter gemessen. Anschließend werden nach 10, 15, 16, 17, 18 und 19 min weitere Blutproben zur Bestimmung der Leukozytenzahl gezogen. Dann werden sofort 500 mg Immunglobulin/kg Körpergewicht iv. anwendbaren Immunglobulins-G innerhalb von 90 sec intraarteriell injiziert. 1, 2, 3, 4, 5, 7, 10, 15 und 20 min nach Injektion werden weitere Blutproben gezogen. Nach 20 min werden 50 mg Immunglobulin/kg Körpergewicht der Standardsubstanz innerhalb von 90 sec injiziert. Blutproben werden wieder nach 1, 2, 3, 4, 5, 7, 10, 15 und 20 min gezogen.

Die maximale Senkung der Leukozytenzahl wird durch Bestimmung von Blutproben, die 1, 2, 3, 4, 5, 7, 10, 15 und 20 min nach der Injektion der Probe gezogen werden, bestimmt.

Die leukopenische Wirkung von iv. anwendbarem Immunglobulin-G wird so ermittelt, daß 500 mg/kg Körpergewicht Hund injiziert werden und der durchschnittliche prozentuelle Leukozytenabfall bei vier Hunden mit der leukopenischen Wirkung von im. anwendbarer Standardsubstanz verglichen wird.

Die Bestimmung der bronchospastischen (atemdrucksteigernden) Wirkung beim Meerschweinchen erfolgt in folgender Weise :

Versuchstieren (Meerschweinchen männlichen Geschlechts) wird nach Narkotisierung die Luftröhre im Kehlkopfbereich präpariert. Nach Intubation wird das Versuchstier mittels eines Beatmungsgerätes mit einem dem Körpergewicht des Tieres entsprechenden Atemvolumen bei einer Atemfrequenz von 80/min beatmet. Danach wird die Arterie carotis vom gleichen Hautschnitt aus freipräpariert. Nach intraarterieller Injektion der Testsubstanz wird der Atemdruck laufend gemessen.

Für den Test werden laborgezüchtete Meerschweinchen männlichen Geschlechts mit einem Körpergewicht zwischen 500 und 700 g verwendet. Pro Testsubstanz werden vier qualifizierte Meerschweinchen benötigt, die bei intraarterieller Applizierung von standardisiertem im. anwendbarem Immunglobulin (Standardsubstanz) einen Atemdruckanstieg um mindestens 30 % bei einer Dosierung von 50 mg/kg Körpergewicht zeigen. Meerschweinchen, die auf die Standardsubstanz keine Reaktion zeigen, können für die Vergleichstests nicht herangezogen werden.

Von der Standardsubstanz werden 160 mg in 1 ml Aqua ad iniectabilia gelöst und mit isotoner NaCl-Lösung auf 16,7 mg/ml verdünnt. Das aufgelöste Material wird innerhalb von vier Stunden verwendet.

Das erfindungsgemäß iv. anwendbare Immunglobulin-G wird mit Aqua ad iniectabilia so gelöst, daß 1 ml 165 mg Protein enthält. Das aufgelöste Material wird innerhalb von vier Stunden verwendet.

Die Tiere werden narkotisiert ; dann wird die Luftröhre im Kehlkopfbereich präpariert, und eine Trachealkanüle wird eingebunden. Mittels eines Beatmungsgerätes wird das Versuchstier mit einem dem Körpergewicht entsprechenden Atemvolumen bei einer Atemfrequenz von 80/min beatmet. Als Beatmungsgerät wird eine Harvard Pumpe Type 681 verwendet.

Danach wird die Arterie carotis vom gleichen Hautschnitt aus freipräpariert und ein Katheter eingebunden. Die Beatmungsdruckregistrierung erfolgt über einen Drucktransducer, der an den Beatmungsschlauch mit Hilfe eines T-Stückes angeschlossen ist.

Nach der Präparation wird zumindest 10 min gewartet, um stabile Ausgangswerte zu erreichen. Danach wird der Nullpunkt festgestellt und nach weiteren zwei bis drei Minuten 150 mg Immunglobulin/kg Körpergewicht iv. anwendbares Immunglobulin-G innerhalb von 90 s intraarteriell über den Katheter injiziert.

Nach 20 min werden 50 mg Standardsubstanz/kg Körpergewicht innerhalb von 90 s intraarteriell injiziert.

Der maximale Atemdruckanstieg während der auf die Injektion der Probe folgenden 20 min wird bestimmt und auf den Ausgangsmittelwert bezogen.

Die bronchospastische Wirkung von iv. anwendbarem Immunglobulin-G wird so ermittelt, daß 500 mg/kg Körpergewicht Meerschweinchen intraarteriell injiziert werden, und der durchschnittliche prozentuelle Atemdruck bei vier Meerschweinchen mit der atemdrucksteigernden Wirkung von im. anwendbarem Immunglobulin-G, Standardsubstanz, verglichen wird.

Erfindungsgemäße Immunglobulin-G-hältige Fraktionen, die in optimaler Weise nebenwirkungsfrei sind, können dadurch hergestellt werden, daß eine aus menschlichem oder tierischem Blut gewonnene Fraktion mit an wasserunlösliches Trägermaterial gebundenen Pankreasenzymen, wie Trypsin oder Chymotrypsin oder Pankreasprotease, behandelt wird und die behandelte Fraktion gegebenenfalls einer weiteren Fraktionierung und Konzentrierung unterworfen wird.

Hierbei wird vorteilhaft als wasserunlösliches Trägermaterial Sepharose 4 B-Gel® verwendet.

Das gereinigte Immunglobulin kann aus der mit an wasserunlösliches Trägermaterial gebundenen Enzymen behandelten immunglobulinhältigen Fraktion, gegebenenfalls nach Entfernen unerwünschter Begleitstoffe, mit Proteinfällungsmitteln ausgefällt und zu einem Endprodukt verarbeitet werden.

Im besonderen hat sich eine Kombination der folgenden Reinigungs- und Konzentrationsmaßnahmen bewährt :

daß aus menschlichem oder tierischem Plasma durch Behandeln mit Äthanol bei einer Temperatur von unter 0 °C ein immunglobulinhältiger Niederschlag ausgefällt wird ;

daß der Niederschlag mittels einer Pufferlösung extrahiert und aus der erhaltenen Lösung durch neuerliche Behandlung mit Äthanol ein pastenförmiges Immunglobulinkonzentrat gewonnen wird ;

daß dieses Konzentrat durch Dialyse gereinigt wird ;

daß die so gereinigte immunglobulinhältige Fraktion mit einem immobilisierten Enzym aus der Gruppe Trypsin, Chymotrypsin oder Pankreasprotease bei erhöhter Temperatur von etwa 37 °C behandelt wird ;

daß aus der so behandelten Fraktion gereinigtes, im wesentlichen aus IgG bestehendes Immunglobulin mittels eines Proteinfällungsmittels, vorzugsweise Polyäthylenglykol, ausgefällt wird und

daß die Fällung gelöst, die Lösung sterilfiltriert und schließlich lyophilisiert wird.

Die Herstellung der erfindungsgemäßen Fraktionen wird durch die folgenden Arbeitsvorschriften und Beispiele näher erläutert :

Arbeitsvorschriften zur Bereitung eines immobilisierten Enzyms

Arbeitsvorschrift 1

1 l Sepharose 4 B-Gel® (Pharmacia) wird nach einer Waschung mit 4 l destilliertem Wasser mit 200 g Bromcyan, die in 100 ml Acetonitril gelöst wurden, bei einem pH-Wert von 11,0 versetzt. Das Reaktionsgemisch wird durch ein Eisbad gekühlt. Nach Entfernung der flüssigen Phase wird das Gel mit 800 mg Trypsin (Sigma), das in 1 l 0,2 molarem $NaHCO_3$ gelöst wurde, versetzt. Das nicht gebundene Trypsin wird vom Trypsin, das an das Gel gebunden ist, durch Filtrieren getrennt.

Nachdem das Gel-Trypsin mit 1 l einer 1 molaren Glycinlösung versetzt wurde, wird es gründlich mit 0,2 molarer $NaHCO_3$-Lösung proteinfrei gewaschen. Schließlich wird das Gel-Trypsin in 1 l 0,9 %iger NaCl-Lösung suspendiert — es ist gebrauchsfertig für die Inkubation mit einer Immunglobulinfraktion.

Arbeitsvorschrift 2

Das unlösliche Enzym wird in der gleichen Weise wie in Arbeitsvorschrift 1 hergestellt, statt Trypsin wird Pankreasprotease (Merck) genommen.

Arbeitsvorschrift 3

Arbeitsvorschrift 1 wird wiederholt, statt Trypsin wird Alpha-Chymotrypsin (Sigma) verwendet.

Beispiele zur Herstellung der Immunglobulin-G-hältigen Fraktion

### Beispiel 1

Menschliches Blutplasma wird mit 8 % Äthanol versetzt, bei einem pH-Wert von 7,2 und einer Temperatur von — 2 °C. Nach Abtrennen des Präzipitates wird die Äthanolkonzentration auf 25 % erhöht und gleichzeitig die Temperatur auf — 6 °C gesenkt. Der ausfallende Niederschlag, der Immunglobulin enthält, wird weiter gereinigt durch eine Extraktion mit einem Phosphat-Acetat-Puffer und er wird mit 12 % Äthanol bei einem pH-Wert von 5,4 und einer Temperatur von — 2 °C versetzt.

Der Niederschlag (der Alpha- und Betaglobulin enthält) wird verworfen. Die Äthanolkonzentration des Überstandes wird, bei einem pH-Wert von 7,2 und einer Temperatur von — 10 °C, auf 25 % erhöht. Das ausgefallene, pastenförmige Immunglobulin wird gesammelt und das Äthanol durch Dialyse entfernt.

Danach wird zum Dialysat 170 g/l Ammoniumsulfat bei einem pH-Wert von 6,25 zugesetzt, das Präzipitat wird abgetrennt und verworfen. Zum Überstand wird bei einem pH-Wert von 7,2 weiteres Ammoniumsulfat bis zu einer Konzentration von 280 g/l zugefügt. Der Niederschlag wird in Wasser gelöst und zur Entfernung des Ammoniumsulfates dialysiert.

Nach der Dialyse wird die Ionenstärke der Immunglobulinlösung auf 0,15 gestellt.

100 g Immunglobulin werden mit 30 ml nach Arbeitsvorschrift 1 immobilisiertem Trypsin hergestellt und bei 37 °C 72 Stunden lang behandelt. Nach Entfernung des Gel-Trypsins wird das behandelte Immunglobulin durch 135 g/l Polyäthylenglykol 4 000 ausgefällt. Das Präzipitat wird in 0,9 % NaCl aufgelöst, sterilfiltriert, abgefüllt und durch Gefriertrocknung haltbar gemacht.

### Beispiel 2

Die Gewinnung der immunglobulinhältigen Fraktion erfolgt in gleicher Weise wie in Beispiel 1.

Die Inkubation wird mit der immobilisierten, nach Arbeitsvorschrift 2 hergestellten Pankreasprotease durchgeführt. 100 g Immunglobulin werden mit 70 ml gelförmiger immobilisierter Pankreasprotease behandelt und bei 37 °C 70 Stunden gehalten. Nach Entfernung des Gels wird der Überstand mit 75 g/l Polyäthylenglykol 4 000 versetzt und der Verunreinigungen enthaltende Niederschlag verworfen.

Zum Überstand wird weiteres Polyäthylenglykol 4 000 bis zu einer Endkonzentration von 85 g/l zugesetzt. Der gebildete Niederschlag wird verworfen.

Durch Erhöhung der Polyäthylenkonzentration auf 135 g/l wird das gereinigte Immunglobulin ausgefällt und wie in Beispiel 1 haltbar gemacht.

### Beispiel 3

Die Gewinnung der Immunglobulin-G-hältigen Fraktion wird in gleicher Weise, wie in Beispiel 1 beschrieben, vorgenommen, jedoch wird die Behandlung mit immobilisiertem Alpha-Chymotrypsin bei 37 °C 72 Stunden durchgeführt.

Die Kennwerte der nach den Beispielen 1 bis 3 hergestellten erfindungsgemäßen Immunglobulin-G-hältigen Fraktionen, nämlich die Gehalte an monomeren IgG-Molekülen, die Gehalte an funktionell intakten IgG-Molekülen, die antikomplementäre Aktivität sowie der Gehalt an Gammaglobulin bei elektrophoretischer Auftrennung wurden wie nachstehend beschrieben bestimmt. Diese Werte sind in den folgenden Tabellen sowie in dem beiliegenden Diagramm I erläutert :

Das Diagramm I veranschaulicht eine Elutionskurve unter den angegebenen Bedingungen zwischen 150 und 400 ml sowie das relative Elutionsvolumen $V_e/V_o$. Die Kurve gibt den Proteingehalt der einzelnen Fraktionen wieder, gemessen bei einer Extinktion von 280 nm.

In der folgenden Tabelle 1 ist das $V_e/V_o$-Verhältnis nach der durchgeführten Gelpermeationschromatographie für die einzelnen, von der Kurve erfaßten Bereiche angegeben. Wie ersichtlich, ist das $V_e/V_o$-Verhältnis im Bereich von 1,30 bis 2,20 über 90 %.

Tabelle 1
Gelpermeationschromatographie

| | $V_e/V_o$ 1,0 - 1,29 | 1,30 - 2,20 | 2,21 - 2,70 |
|---|---|---|---|
| Beispiel 1 | Spuren | 92,4 % | 7,6 % |
| Beispiel 2 | - | 93,2 % | 6,8 % |
| Beispiel 3 | - | 91,8 % | 8,2 % |

In der Tabelle 2 ist das Verhältnis der an Protein A gebundenen und der nichtgebundenen IgG-Moleküle dargestellt, wobei die gebundenen den funktionell intakten entsprechen. Wie ersichtlich, ist der Gehalt der funktionell intakten IgG-Moleküle in der gesamten Fraktion über 90 %.

Tabelle 2
Affinitätschromatographie mit Protein A-Sepharose

| | % von Fraktion $V_e/V_o$ 1,30 - 2,30 | | % von gesamter Immunglobulin-G-hältiger Fraktion |
|---|---|---|---|
| | nichtgebunden | gebunden | |
| Beispiel 1 | 1,5 | 98,5 | 91,0 |
| Beispiel 2 | 0,9 | 99,1 | 92,3 |
| Beispiel 3 | 1,2 | 98,8 | 90,7 |

In Tabelle 3 sind die Werte für die antikomplementäre Aktivität und für die Elektrophorese angegeben, woraus ersichtlich ist, daß bei den Präparationen nach allen Beispielen Werte der antikomplementären Aktivität von mehr als 50 mg Immunglobulin-G-hältiger Fraktion zur Neutralisierung einer C'H-50-Einheit benötigt und elektrophoretisch bestimmte Werte an reinem Gammaglobulin von mehr als 97 % erhalten wurden.

Tabelle 3

| | Antikomplementäre Aktivität | Elektrophorese |
|---|---|---|
| Beispiel 1 | > 50 mg/C'H-50 | > 97,0 % reines Gammaglobulin |
| Beispiel 2 | > 50 mg/C'H-50 | > 97,0 % reines Gammaglobulin |
| Beispiel 3 | > 50 mg/C'H-50 | > 97,0 % reines Gammaglobulin |

Die pharmakologischen Merkmale bzw. Kennwerte der nach den Beispielen 1 bis 3 hergestellten erfindungsgemäßen Immunglobulin-G-hältigen Fraktionen, nämlich die vasoaktive und leukopenische Wirkung im Hundetest und die bronchospastische Wirkung im Meerschweinchentest, wurden wie im Vorherstehenden beschrieben bestimmt ; diese Werte sind aus den folgenden Tabellen zu ersehen :

Tabelle 4

Durchschnittsblutdruck von vier Hunden in % des Ausgangswertes nach Injizierung von 500 mg der erfindungsgemäßen Präparation pro kg Körpergewicht

| | Systolischer Blutdruck | Diastolischer Blutdruck |
|---|---|---|
| Präparation nach Beispiel 1 | 91 % | 86 % |
| Präparation nach Beispiel 2 | 93 % | 89 % |
| Präparation nach Beispiel 3 | 81 % | 79 % |

Tabelle 5

Durchschnittsleukozytenzahl von vier Hunden in % des Ausgangswertes nach Injizierung von 500 mg der erfindungsgemäßen Präparation pro kg Körpergewicht

| | |
|---|---|
| Beispiel 1 | 73 % |
| Beispiel 2 | 62 % |
| Beispiel 3 | 52 % |

Tabelle 6

Durchschnittsatemdruckanstieg von vier Meerschweinchen in % des Ausgangswertes nach Injizierung von 500 mg der erfindungsgemäßen Präparation pro kg Körpergewicht

| | |
|---|---|
| Beispiel 1 | 102 % |
| Beispiel 2 | 110 % |
| Beispiel 3 | 125 % |

Die Überlegenheit der erfindungsgemäßen, intravenös anwendbaren Immunglobulin-G-hältigen Fraktionen gegenüber den bekannten, intramuskulär anwendbaren ist mit großer Anschaulichkeit aus den beiliegenden Diagrammen II bis IV zu ersehen.

Im Diagramm II sind die Blutdruckkurven bei systolischer und diastolischer Messung von jeweils vier Hunden aufgezeichnet, wobei auf der Abszisse jeweils die verabreichten Mengen in mg/kg Körpergewicht der Tiere aufgetragen sind. Die voll ausgezeichnete Linie im Bereich von A bis B entspricht dem Verlauf des Blutdrucks bei Verabreichung von erfindungsgemäßen, intravenös anwendbaren Immunglobulin-G-hältigen Präparaten ; der Verlauf der Kurve von A bis C (voll ausgezogene Linie und strichlierte Linie) entspricht dem Verlauf der Blutdruckkurve bei Applikation der im.-Standardsubstanz. Es ist ersichtlich, daß bei einer Applikation der im. anwendbaren Standardsubstanz von 5 mg/kg Körpergewicht der Blutdruck um 30 % abgefallen ist, wogegen bei der Applikation des erfindungsgemäßen Präparates dieser Abfall erst bei einer Dosierung von 500 mg/kg Körpergewicht auftritt, d. h. das erfindungsgemäße intravenös anwendbare Immunglobulin hat eine um das mindestens 100fach geringere vasoaktive Wirkung als die bekannten im. anwendbaren Präparate unter sonst gleichen Bedingungen.

Aus dem Diagramm III ist die leukopenische Wirkung im Hundetest als Durchschnitt von vier Tieren im Vergleich zwischen dem erfindungsgemäßen, iv. anwendbaren Präparat und dem standardisierten, im. anwendbaren Standardpräparat zu ersehen. Die voll ausgezogene Linie im Bereich von A bis B entspricht dem erfindungsgemäßen intravenös anwendbaren Immunglobulin-G-hältigen Präparat, und die Linie von A bis C (voll ausgezogene Linie und strichlierte Linie) dem Verlauf der Leukozytenzahl bei Applikation der im. anwendbaren Standardsubstanz. Es ist ersichtlich, daß bei einer Applikation der im. anwendbaren Standardsubstanz die Leukozyten um 50 % abgefallen sind, wogegen bei der Applikation des erfindungsgemäßen Präparates dieser Abfall erst bei einer Dosierung von 500 mg/kg Körpergewicht auftritt, d. h. das erfindungsgemäße intravenös anwendbare Immunglobulin-G-hältige Präparat hat eine um das mindestens 1 000fach niedrigere leukopenische Wirkung als die bekannten, im. anwendbaren Präparate unter sonst gleichen Bedingungen.

Ähnlich sieht der Vergleich der bronchospastischen Wirkungen im Meerschweinchentest gemäß dem Diagramm IV aus, wobei der Verlauf der Kurve von A bis B (voll ausgezogene Linie) dem erfindungsgemäßen iv. anwendbaren Präparat und der Verlauf der Kurve von A bis C (voll ausgezogene und strichlierte Linie) dem standardisierten im. anwendbaren Präparat entspricht. Der 30 %ige Atemdruckanstieg erfolgt beim bekannten im. anwendbaren Präparat schon bei einer Dosierung von 2 mg/kg Körpergewicht, wogegen der gleiche Anstieg beim iv. anwendbaren Präparat erst bei einer Dosis von 500 mg/kg Körpergewicht erfolgt, d. h. daß die bronchospastische Nebenwirkung bei dem erfindungsgemäßen Präparat um das 250fache geringer ist.

Aufgrund ihrer chemischen Zusammensetzung und ihrer pharmakologischen Eigenschaften sind die erfindungsgemäßen Immunglobulin-G-hältigen Fraktionen in hervorragender Weise zur Verwendung für die Behandlung bzw. für die Herstellung von Präparationen zur Behandlung von primären und sekundären Immundefekten, A- oder Hypogammaglobulinamie, bei Antikörpermangelsyndrom, bei Virusinfektionen oder bakteriellen Infektionen sowie bei Autoimmun- und Immunkomplexerkrankungen geeignet.

**Patentansprüche**

1. Immunglobulin-G-hältige Fraktion aus menschlichem oder tierischem Plasma zur intravenösen Anwendung, dadurch gekennzeichnet, daß sie
mindestens 90 % monomere IgG-Moleküle,
mindestens 95 % Gammaglobuline,
mindestens 90 % funktionell intakte IgG-Moleküle, d. h. mindestens 90 % IgG-Moleküle, die an Protein A von Staphylococcus aureus bindbar sind, enthält, eine antikomplementäre Aktivität von mindestens 40 mg Protein zur Neutralisation einer C'H-50-Einheit aufweist und im wesentlichen frei ist von vasoaktiv und leukopenisch wirksamen sowie von bronchospastischen Substanzen, ausgedrückt durch folgende Merkmale :

a) daß die vasoaktive Wirkung im Hundetest als Durchschnitt bei vier Tieren, d. h. ein Blutdruckabfall von höchstens 30 %, erst bei einer Dosis von mehr als 500 mg/kg Körpergewicht,

b) daß die leukopenische Wirkung im Hundetest als Durchschnitt bei vier Tieren, d. h. ein Abfall der Leukozytenzahl von höchstens 50 %, erst bei einer Dosis von mehr als 500 mg/kg Körpergewicht, und

c) daß die bronchospastische Wirkung im Meerschweinchentest als Durchschnitt bei vier Tieren, d. h. ein Atemdruckanstieg von höchstens 30 %, erst bei einer Dosis von mehr als 500 mg/kg Körpergewicht

feststellbar ist.

2. Verwendung einer Immunglobulin-G-hältigen Fraktion nach Anspruch 1 für die Herstellung von Präparationen für die Behandlung von primären und sekundären Immundefekten, A- oder Hypogammaglobulinamie, bei Antikörpermangelsyndrom, bei Virusinfektionen oder bakteriellen Infektionen sowie bei Autoimmun- und Immunkomplexerkrankungen.

**Claims**

1. Immunoglobulin-G-containing fraction from human or animal plasma for intravenous application, characterised in that it contains :
at least 90 % monomeric IgG molecules,
at least 95 % gammaglobulins,
at least 90 % functionally intact IgG molecules, i. e., at least 90 % IgG molecules capable of binding to protein A of Staphylococcus aureus, it has an anticomplementary activity of at least 40 mg protein for neutralisation of a C'H-50 unit, and it is substantially free of vasoactive and leucopenically active as well as bronchospastic substances expressed by the following characteristic features :

a) that the vasoactive effect, in dog test as an average of four animals, i. e., a blood pressure decrease by 30 % at most, is detectable only at a dose of more than 500 mg/kg body weight,

b) that the leucopenic effect, in dog test as an average of four animals, i. e., a decrease of the leucocyte number of 50 % at most, is detectable only at a dose of more than 500 mg/kg body weight, and

c) that the bronchospastic effect, in guinea pig test as an average of four animals, i. e., an increase in the respiratory pressure by 30 % at most, is detectable only at a dose of more than 500 mg/kg body weight.

2. Use of an immunoglobulin-G-containing fraction according to claim 1 for the production of preparations for the treatment of primary and secondary immune defects, A or hypogammaglobulinaemia, antibody deficiency syndrome, virus infections or bacterial infections as well as autoimmune and immune complex diseases.

**Revendications**

1. Fraction de plasma humain ou animal contenant des immunoglobulines G pour l'utilisation intraveineuse, caractérisée en ce qu'elle contient :
au moins 90 % de molécules d'IgG monomères,
au moins 95 % de gammaglobulines,
au moins 90 % de molécules d'IgG fonctionnellement intactes, c'est-à-dire au moins 90 % de molécules d'IgG qui peuvent être fixées sur la protéine A de Staphylococcus aureus, elle présente une activité anticomplémentaire d'au moins 40 mg de protéine pour la neutralisation d'une unité C'H-50 et elle est pratiquement exempte de substances à activité vasomotrice et leucopénique ainsi que de substances bronchospasmodiques, exprimées par les paramètres suivants :

a) l'activité vasomotrice dans le test sur le chien en moyenne de quatre animaux, c'est-à-dire une chute de la pression sanguine de 30 % au plus, n'est décelable qu'à une dose de plus 500 mg/kg de poids du corps,
b) l'activité leucopénique dans le test sur le chien en moyenne de quatre animaux, c'est-à-dire une chute du nombre de leucocytes de 50 % au plus, n'est décelable qu'à une dose de plus de 500 mg/kg de poids du corps et
c) l'activité bronchospasmodique dans le test sur le cochon d'Inde, en moyenne de quatre animaux, c'est-à-dire une élévation de la pression respiratoire de 30 % au plus, n'est décelable qu'à une de plus de 500 mg/kg de poids du corps.

2. Utilisation d'une fraction contenant des immunoglobulines G selon la revendication 1 pour la fabrication de préparations pour le traitement de déficiences autoimmunes primaires et secondaires, de protéinémie A ou d'hypogammaglobulinémie, dans le syndrome de déficience en anticorps, dans les infections virales ou les infections bactériennes ainsi que dans les maladies auto-immunes et les maladies des complexes immuns.

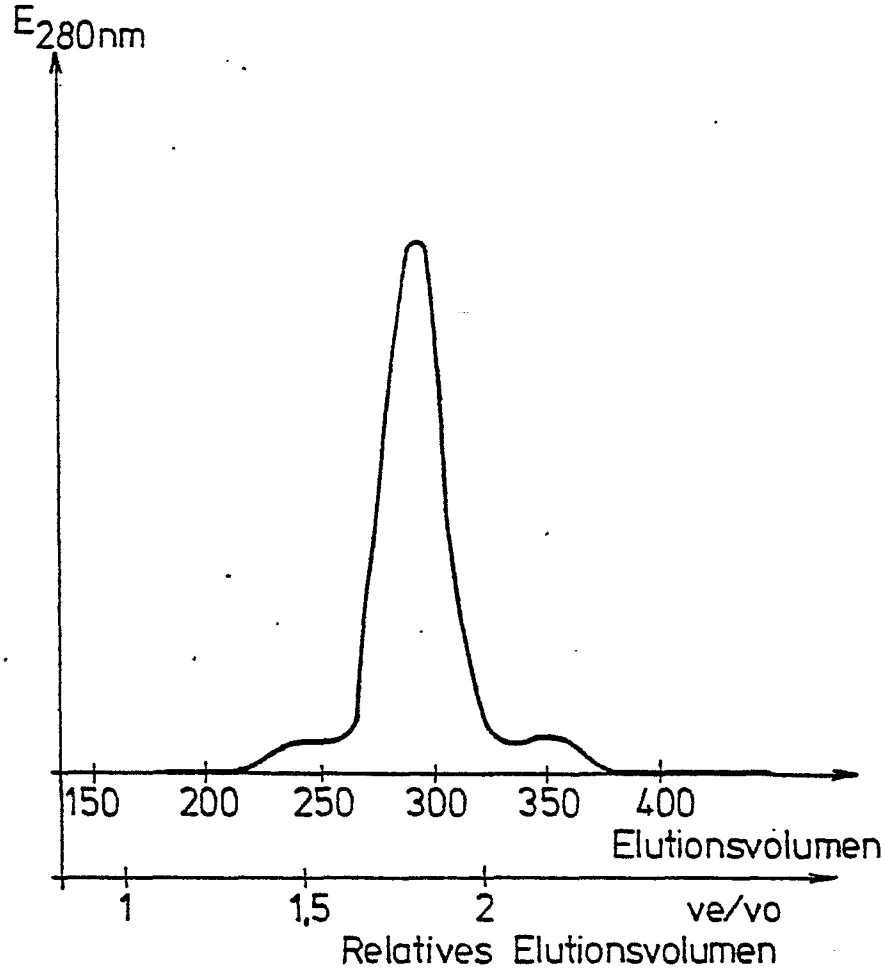
E280nm
150
200
250
300
350
400
Elutionsvolumen
1
1,5
2
ve/vo
Relatives Elutionsvolumen

Diagramm I

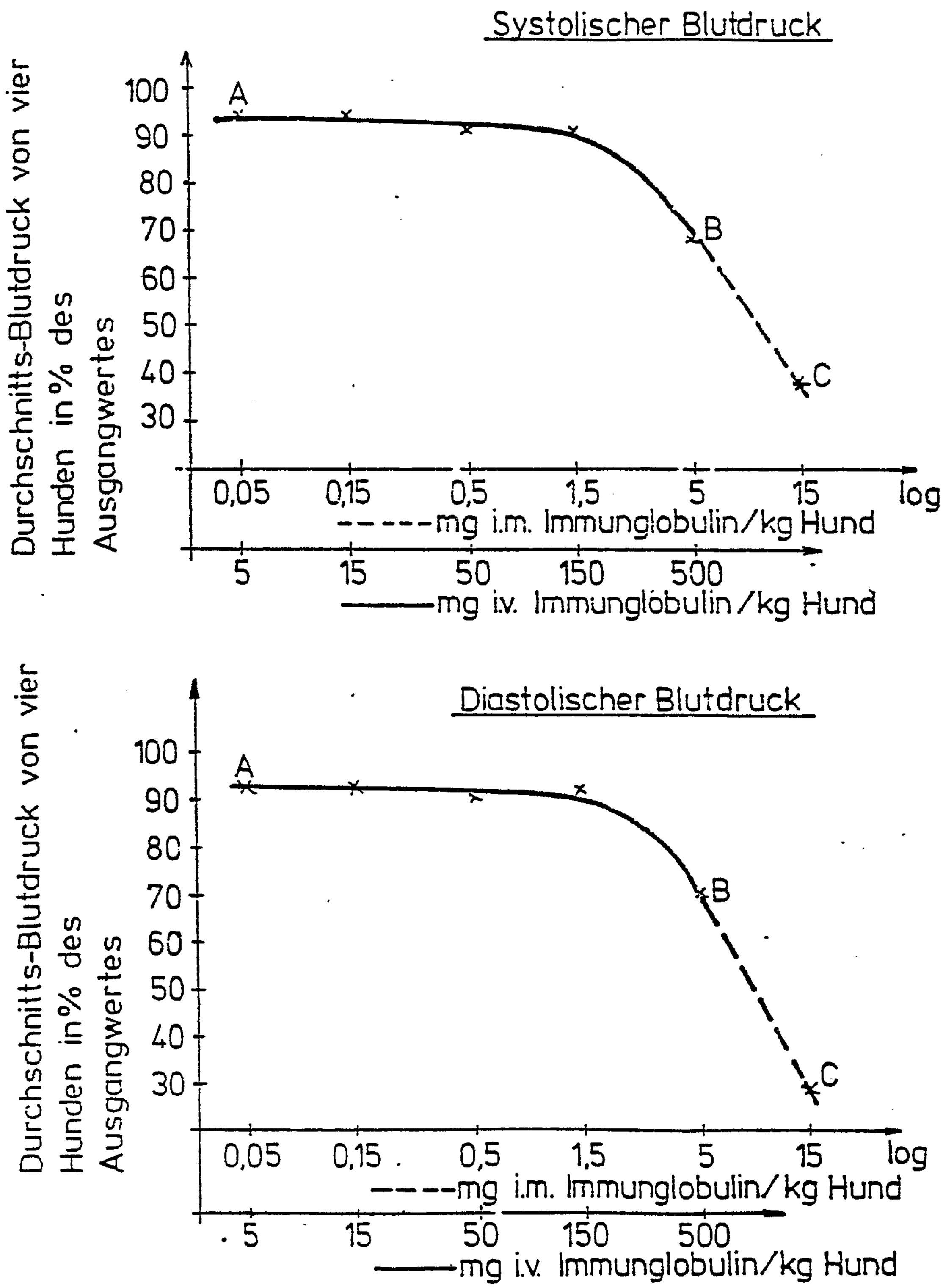
Systolischer Blutdruck
Durchschnitts-Blutdruck von vier Hunden in% des Ausgangwertes
100
90
80
70
60
50
40
30
A
B
C
0,05
0,15
0,5
1,5
5
15
log
-----mg i.m. Immunglobulin/kg Hund
5
15
50
150
500
——mg i.v. Immunglobulin/kg Hund
Diastolischer Blutdruck
Durchschnitts-Blutdruck von vier Hunden in% des Ausgangwertes
100
90
80
70
60
50
40
30
A
B
C
0,05
0,15
0,5
1,5
5
15
log
-----mg i.m. Immunglobulin/kg Hund
5
15
50
150
500
——mg i.v. Immunglobulin/kg Hund

Diagramm II

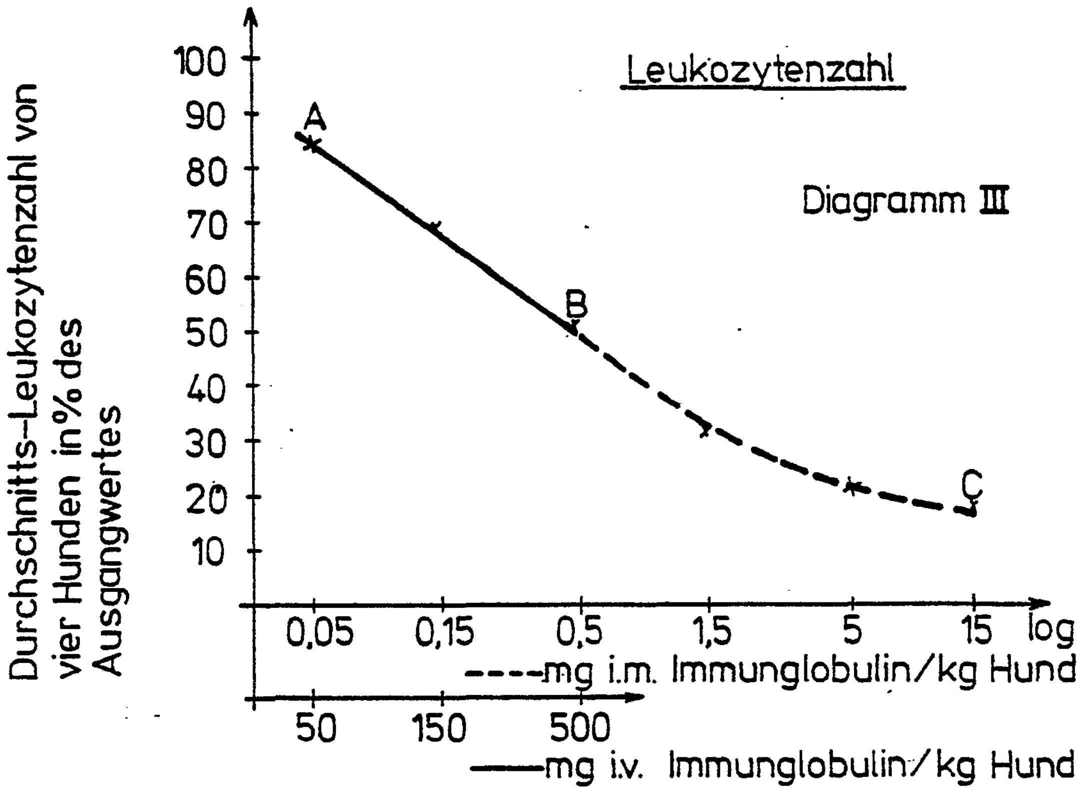
Leukozytenzahl
Diagramm III
Durchschnitts-Leukozytenzahl von vier Hunden in% des Ausgangwertes
100
90
80
70
60
50
40
30
20
10
A
B
C
0,05
0,15
0,5
1,5
5
15
log
----mg i.m. Immunglobulin/kg Hund
50
150
500
——mg i.v. Immunglobulin/kg Hund

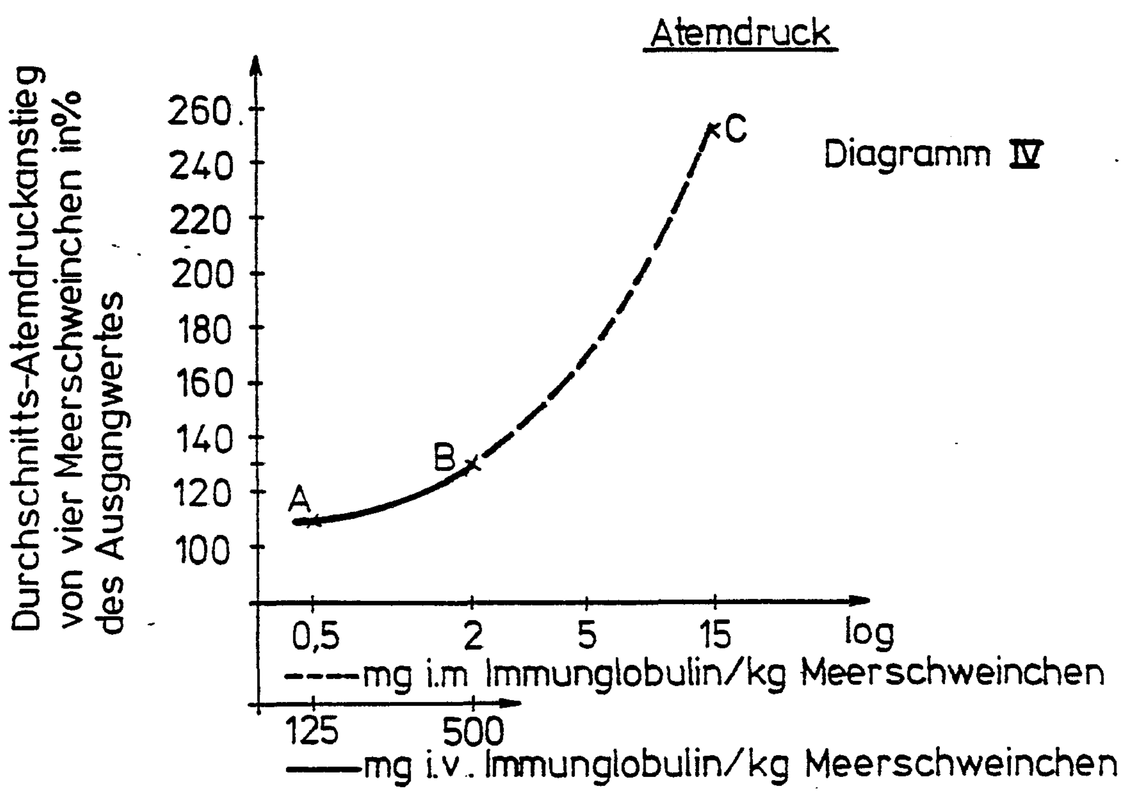
Atemdruck
Diagramm IV
Durchschnitts-Atemdruckanstieg von vier Meerschweinchen in% des Ausgangwertes
260
240
220
200
180
160
140
120
100
A
B
C
0,5
2
5
15
log
----mg i.m Immunglobulin/kg Meerschweinchen
125
500
——mg i.v. Immunglobulin/kg Meerschweinchen